# DEMANDE DE BREVET EUROPEEN

(11) **EP 1 064 928 A1**
(43) Date de publication de la demande: **03.01.2001**
(21) Numéro de dépôt: 00401474.2
(22) Date de dépôt: 25.05.2000
(51) Int. Cl.: A61K 7/48, A61K 7/00, A61K 7/02

(54) **Utilisation dans une composition cosmétique démaquillante ou nettoyante d'au moins un composé fluoré volatil**

(30) Priorité: 30.06.1999 FR 9908373
(71) Demandeur: L'OREAL, 75008 Paris (FR)
(72) Inventeur: Lorant, Raluca, 94320 Thiais (FR)
(74) Mandataire: Dodin, Catherine

(57) **Abrégé**

L'invention concerne l'utilisation pour le démaquillage ou le nettoyage de la peau d'une composition anhydre ou aqueuse, contenant au moins un composé fluoré volatil, en une proportion d'au moins 0,5 % en poids par rapport au poids total de la composition.

La composition se présente sous forme d'une huile, d'une crème ou d'un lait démaquillant.

La présence du composé fluoré volatil apporte une amélioration des propriétés démaquillantes ainsi qu'un effet de fraîcheur après application.

## Description

La présente invention a pour objet l'utilisation d'une composition cosmétique, anhydre ou aqueuse, pour le démaquillage ou le nettoyage de la peau, notamment du visage, contenant au moins un composé fluoré volatil. Plus particulièrement, la présente invention a pour objet l'utilisation d'une composition sous forme d'une huile, d'une crème ou d'un lait démaquillant. L'invention se rapporte également à une composition cosmétique sous forme d'une émulsion eau-dans-l'huile ou huile-dans-l'eau. L'invention a en outre pour objet un procédé de démaquillage ou de nettoyage de la peau notamment du visage.

Le démaquillage consiste essentiellement à dissoudre et à éliminer toutes les traces de maquillage ainsi que les impuretés accumulées sur le visage provenant de la pollution atmosphérique. Il est important, lors du démaquillage, de bien nettoyer la peau sans l'agresser.
Le démaquillage est le plus souvent réalisé à l'aide de produits fluides à base d'agents tensioactifs généralement en solution aqueuse, ou sous forme d'une émulsion. Si ces produits démaquillants permettent une bonne élimination des maquillages classiques dits "non-waterproof", ils présentent néanmoins l'inconvénient d'assécher la peau par élimination de son film hydrolipidique naturel et de provoquer certaines irritations cutanées, notamment des parties les plus sensibles du visage, telles que les paupières, le contour des yeux, et les lèvres. Les produits démaquillants à base de tensioactifs détergents sont donc vivement déconseillés pour le démaquillage des peaux sensibles et délicates. Par contre, lorsque les maquillages sont du type dit "waterproof" ceux-ci nécessitent l'utilisation de produits démaquillants spécifiques qui sont constitués d'une huile telle que l'huile de vaseline en association par exemple avec des esters gras. Ces produits démaquillants présentent toutefois de tels désagréments à l'application (sensation grasse sur la peau) que lorsqu'ils ne sont pas indispensables, on préfère employer les produits démaquillants classiques, mais ceux-ci manquent alors d'efficacité.

Après de nombreuses études sur divers types de composés, on a constaté de façon surprenante et inattendue, que l'utilisation de certains composés fluorés volatils dans de telles compositions permettait d'apporter des propriétés démaquillantes particulièrement performantes ainsi qu'un effet immédiat de fraîcheur après application.
De plus, on a constaté que lorsque ces compositions démaquillantes étaient sous forme d'une émulsion, celles-ci pouvaient être avantageusement formulées en présence d'une faible proportion d'un agent tensioactif détergent sans affecter pour autant leur pouvoir démaquillant, ce qui permettait dès lors d'obtenir de meilleures propriétés cosmétiques d'agrément et de confort. Un autre avantage particulièrement appréciable de ces nouvelles compositions réside dans le fait qu'elles sont susceptibles d'éliminer les différents types de maquillage ("waterproof' ou "non-waterproof"), ainsi que les maquillages dits "anti-transfert" et ceci dans des conditions très satisfaisantes.

La présente invention a donc pour objet l'utilisation pour le démaquillage ou le nettoyage de la peau du corps ou du visage, ou des lèvres, d'une composition anhydre ou aqueuse, caractérisée par le fait que ladite composition contient au moins un composé fluoré volatil, en une proportion d'au moins 0,5 % en poids par rapport au poids total de ladite composition.
Un autre objet de l'invention est une composition cosmétique susceptible d'être utilisée pour le démaquillage ou le nettoyage de la peau du corps ou du visage, ou des lèvres, se présentant sous la forme d'une émulsion comprenant une phase grasse, une phase aqueuse et éventuellement un agent tensioactif, caractérisée par le fait que ladite phase grasse comprend au moins 5 % en poids d'un composé fluoré volatil, et est présente en une proportion comprise entre 2 et 70 % en poids, et que la phase aqueuse est présente en une proportion comprise entre 30 et 98 % en poids par rapport au poids total de la composition.

Selon l'invention, le composé fluoré volatil est généralement présent dans la composition en une proportion d'au moins 5 % et de préférence entre 5 et 80 % et de préférence encore entre 8 et 70 % en poids par rapport au poids total de la composition.

Les composés fluorés volatils utilisables selon l'invention qui peuvent être de type huileux, sont de préférence choisi parmi, seuls ou en mélange :
1°) les perfluorocycloalkyles répondant à la formule (I) suivante : 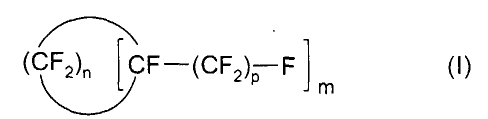 dans laquelle :
   n est 3, 4 ou 5
   m est 1 ou 2, et
   p est 1,2 ou 3
   sous réserve que lorsque m=2, les groupements ne sont pas nécessairement en alpha, l'un par rapport à l'autre ;
2°) les perfluoroalcanes répondant à la formule (II) suivante :

   CF₃-(CF₂)ₘ-CF₂X (II)

   dans laquelle :
   m est 2 à 8, et
   X représente Br ou F ;
3°) les fluoroalkyles ou hétérofluoroalkyles répondant à la formule (III) suivante :

   CH₃-(CH₂)ₙ-[Z]ₜ-X-CF₃ (III)

   dans laquelle :
   t est 0 ou 1,
   n est 0, 1, 2 ou 3,
   X est un radical perfluoroalkyle, linéaire ou ramifié, ayant de 2 à 5 atomes de carbone, et
   Z est O, S ou NR, R étant un hydrogène, un radical -(CH₂)ₙ-CF₃ ou -(CF₂)ₘ-CF3, m étant 2, 3, 4 ou 5, et
4°) les dérivés de perfluoromorpholine répondant à la formule (IV) suivante : 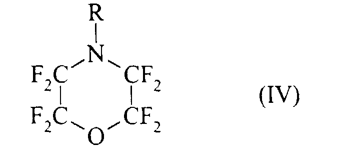 dans laquelle :
   R est un radical perfluoroalkyle en C₁-C₄.

Parmi les perfluorocycloalkyles de formule (I), on peut notamment citer le perfluorométhylcyclopentane et le perfluoro-1,3 diméthylcyclohexane, qui sont vendus sous les dénominations de "FLUTEC PC1®" et "FLUTEC PC₃®" par la Société BNFL FLUOROCHEMICALS Ltd., et le perfluoro-1,2-diméthylcyclobutane. Parmi les perfluoroalcanes de formule (II), on peut citer, entre autre, le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société ATOCHEM.
Parmi les composés fluorés de formule (III), on peut par exemple citer le nonafluorométhoxybutane vendu sous la dénomination "MSX 4518®" par la Société 3M et le nonafluoroéthoxyisobutane.
Enfin, parmi les dérivés de perfluoromorpholine de formule (IV), on peut citer par exemple la 4-trifluorométhyl perfluoromorpholine vendue sous la dénomination "PF 5052®" par la Société 3M.

Les composés fluorés tels que décrits ci-dessus sont par ailleurs caractérisés par leur densité élevée, généralement supérieure à 1, de préférence supérieure à 1,2, par une pression de vapeur saturante, à 25°C, au moins égale à 50 Pa et par un point d'ébullition généralement compris entre 25 et 65°C.

Lorsque selon l'invention on utilise une composition anhydre, il s'agit essentiellement d'une huile, le composé fluoré volatil étant présent soit à l'état soluble, soit à l'état finement dispersé dans au moins une huile cosmétique.
Parmi les huiles non fluorées, miscibles avec le composé fluoré volatil, on peut notamment citer les polysiloxanes en particulier les PDMS notamment volatils tels que le cyclopentasiloxane et le cyclohexasiloxane, les huiles hydrocarbonées volatiles et notamment les isoparaffines en C₁₁-C₁₃ ainsi que l'isododécane.
Parmi les huiles non fluorées, non miscibles avec le composé fluoré volatil, on peut notamment citer les esters d'acides gras, comportant au moins 6 atomes de carbone, obtenus de préférence à partir d'un alcool, linéaire ou ramifié, en C₁-C₁₇ et d'un acide gras, linéaire ou ramifié, en C₆-C₂₂, de préférence un mono ou di-acide gras.
Parmi ces esters, on peut mentionner le palmitate d'éthyl-2-hexyle, le myristate d'éthyl-2-hexyle, le palmitate d'isopropyle, le myristate d'isopropyle, l'adipate de di-isopropyle, l'adipate de di-octyle, l'hexanoate d'éthyl-2-hexyle, le laurate d'éthyle, le myristate de méthyle, l'octanoate d'octyldodécyle, le néopentanoate d'isodécyle, le myristate d'éthyle, le propionate de myristyle, l'éthyl-2-hexanoate d'éthyl-2-hexyle, l'octanoate d'éthyl-2-hexyle, le caprate/caprylate d'éthyl-2-hexyle, le palmitate de méthyle, le myristate de butyle, le myristate d'isobutyle, le palmitate d'éthyle, le laurate d'isohexyle, le laurate d'hexyle, l'isostéarate d'isopropyle et leurs mélanges.
Selon cette forme de réalisation, la concentration en composé fluoré volatil est généralement comprise entre 20 et 80 % et de préférence entre 30 et 65 % par rapport au poids total de la composition sous forme d'huile.

Lorsque l'on utilise selon l'invention une composition cosmétique aqueuse, celle-ci se présente sous forme d'émulsions multiples mais de préférence sous forme d'une émulsion eau-dans-l'huile ou huile-dans-l'eau contenant une phase grasse, une phase aqueuse et éventuellement un agent tensio-actif, la phase grasse contenant le composé fluoré volatil.
Les émulsions constituent une forme de réalisation préférée pour le démaquillage et le nettoyage de la peau et contiennent de préférence, par rapport au poids total de l'émulsion, de 2 à 70 % mais de préférence de 5 à 30 % en poids de phase grasse, et de 30 à 98 % mais de préférence de 70 à 95 % en poids de phase aqueuse.
La proportion en composé fluoré volatil dans la phase grasse est généralement comprise entre 10 et 100 %, mais de préférence entre 30 et 95 % et de préférence encore entre 40 et 80 % en poids par rapport au poids total de ladite phase.

Lorsque la phase grasse des émulsions selon l'invention n'est pas constituée du seul composé fluoré volatil, celle-ci se présente alors sous forme d'un mélange avec au moins une huile cosmétique non fluorée, miscible ou non miscible, avec le composé fluoré volatil, en une proportion comprise entre 0,5 et 60 %, de préférence entre 1 et 40 % en poids par rapport au poids total de ladite phase.
Les huiles non fluorées, miscibles ou non miscibles, avec le composé fluoré volatil de la phase grasse des émulsions, peuvent être notamment choisies parmi celles précédemment énumérées.
La phase aqueuse des émulsions peut également renfermer tout ingrédient hydrosoluble communément utilisé dans les émulsions cosmétiques. On citera par exemple, les agents hydratants tels que le glycérol et le propylène glycol. Comme indiqué précédemment, les émulsions selon la présente invention présentent un excellent pouvoir démaquillant intrinsèque, en l'absence d'agent tensioactif détergent. Toutefois, elles peuvent en contenir une faible proportion généralement présente en vue d'assurer un stabilité satisfaisante de l'émulsion. Selon cette forme de réalisation, l'agent tensioactif est généralement présent en une proportion comprise entre 0,2 et 8 % en poids en matière active par rapport au poids total de l'émulsion.
Lorsque les compositions démaquillantes se présentent sous forme d'une émulsion huile-dans-l'eau, l'agent tensioactif est de préférence un agent tensioactif anionique ou amphotère comprenant au moins un groupement phosphate, sulfate ou acétylméthyltaurate tel que ceux décrits dans la demande FR-2 745 715. Parmi ces agents tensioactifs, on peut citer ceux commercialisés sous les dénominations "PECOSIL PS-100®, PECOSIL PS-200®", et "PECOSIL WDS-100®" par la Société PHOENIX CHEMICAL.
Lorsque les émulsions démaquillantes sont du type eau-dans-l'huile, elles contiennent alors de préférence des agents tensioactifs siliconés, en particulier ceux appartenant à la famille des alkyl- ou alcoxydiméthicones copolyols ou encore des diméthicones copolyols tels que ceux décrits dans la demande FR-2 701 845. Parmi ces tensioactifs, on peut citer notamment ceux vendus sous les dénominations de "Abil WE09®", "Abil WS08®", ou "Abil EM90®" par la Société GOLDSCHMIDT, de "Q2 5200®" ou "Q2 3225®" par la Société Dow CORNING et de "218-1138®" ou de "SF 1228®" par la Société GENERAL ELECTRIC.
Les émulsions selon l'invention sont plus ou moins fluides et ont plus particulièrement l'aspect d'un lait ou d'une crème. La préparation des laits ou crèmes démaquillantes ou nettoyantes est généralement facilitée par l'utilisation d'un agent épaississant qui est alors présent en une proportion de préférence comprise entre 0,1 et 5 % en poids par rapport au poids total de la composition sous forme de lait ou de crème.
L'agent épaississant peut être notamment choisi parmi :
- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose, et la carboxy-méthylcellulose ;
- les polymères synthétiques comme les acides polyacryliques tels que les polymères poly(méth)acrylates de glycéryle vendus sous les dénominations "HISPAGEL®", "LUBRAGEL" ou "SEPIGEL 305®" respectivement par les Sociétés HISPANO QUIMICA, GUARDIAN et SEPPIC, la polyvinylpyrrolidone, l'alcool polyvinylique, les polymères réticulés d'acrylamide et d'acrylate d'ammonium vendus sous les dénominations "PAS 5161®" ou "BOZEPOL®" par la Société HOECHST, les polymères réticulés d'acrylamide et de chlorure de méthacryloyloxyéthyltriméthylammonium vendus sous la dénomination "SALCARE SC 92®" par la Société ALLIED COLLOIDS, et le silicate de magnésium et d'aluminium.
On utilise de préférence, en tant qu'agent épaississant, les polymères réticulés d'acrylamide et d'acide 2-acrylamido 2-méthylpropane sulfonique, également désignés polymères d'AMPS, partiellement ou totalement neutralisés et vendus sous la dénomination de "HOSTACERIN AMPS®" par la Société CLARIANT. Ceux-ci sont notamment décrits dans la demande EP-503 853, dont le contenu est incorporé par référence dans la présente description.

Les compositions selon l'invention telles que définies ci-dessus peuvent comprendre en outre tout ingrédient cosmétique conventionnel, tel que par exemple des conservateurs, des antioxydants, des parfums, des colorants solubles ou des principes actifs ayant notamment une activité émolliente, régénérante, décongestionnante, anti-inflammatoire, éclaircissante, détoxifiante, cicatrisante ou adoucissante.

La présente invention se rapporte par ailleurs à un procédé de démaquillage ou de nettoyage de la peau du corps ou du visage, ou des lèvres, qui consiste à appliquer, de préférence avec les doigts, un coton ou un tissu de papier, une quantité suffisante d'une composition telle que définie ci-dessus, sur les parties de la peau à démaquiller ou à nettoyer, à masser ensuite légèrement de façon à détacher un maximum d'impuretés et du produit de maquillage, et à éliminer la composition à l'aide d'un coton éventuellement imbibé d'eau.

On va maintenant donner à titre d'illustration plusieurs exemples de compositions démaquillantes ou nettoyantes pour le visage selon l'invention.

### EXEMPLE 1 : Lotion huileuse démaquillante

On prépare une lotion anhydre huileuse pour le démaquillage en procédant au mélange des ingrédients suivants :
- Nonafluorométhoxybutane 40,0 %
- Tétradécafluorohexane 20,0 %
- Cyclohexasiloxane 40,0 %

Cette lotion, homogène et limpide, permet par application à l'aide d'un coton sur les parties du visage à démaquiller, une excellente élimination du maquillage tout en apportant fraîcheur et douceur.

### EXEMPLE 2 : Lait démaquillant

On prépare un lait démaquillant en procédant au mélange des ingrédients suivants :

### A - Phase grasse

- Nonafluorométhoxybutane 10,0 %

### B - Phase aqueuse

- Gomme de xanthane 0,2 %
- Sodium diméthicone copolyol acétyl méthyl laurate vendu sous la dénomination commerciale "PECOSIL DCT®" par la Société PHOENIX CHEMICAL 5,4 %
- Conservateurs 0,2 %
- Eau 84,8 %

Ce lait démaquillant s'applique sur l'ensemble du visage, enrobe toutes les traces de maquillage et les impuretés, et laisse une peau nettoyée en profondeur, souple et très fraîche.

### EXEMPLE 3 : Crème démaquillante

On prépare une crème démaquillante en procédant au mélange des ingrédients suivants :

### A - Phase grasse

- Tétradécafluorohexane 10,0 %
- Cyclohexasiloxane 5,0 %

### B - Phase aqueuse

- Polymère d'AMPS vendu sous la dénomination "HOSTACERIN AMPS®" par la Société CLARIANT 2,0 %
- Conservateurs 0,2 %
- Eau 82,8 %
Cette crème, sans tensioactif, est particulièrement adaptée pour les peaux sensibles, car elle dissout en douceur les impuretés et les maquillages "waterproof' et "non-waterproof" tout en respectant l'intégrité du film hydrolipidique cutané.

### EXEMPLE 4 : Emulsion démaquillante

On prépare une émulsion démaquillante en procédant au mélange des ingrédients suivants :

### A - Phase grasse

- Nonafluorométhoxybutane 20,0 %

### B - Phase aqueuse

- Sodium diméthicone copolyol acétyl méthyl laurate vendu sous la dénomination commerciale "PECOSIL DCT®" par la Société PHOENIX CHEMICAL 5,4 %
- Conservateurs 0,2 %
- Gomme de xanthane 0,2 %
- Eau 74,2 %

Par application de cette émulsion à l'aide d'un coton sur les parties du visage à nettoyer, on obtient un excellent démaquillage, la peau restant souple avec une sensation de fraîcheur.

### EXEMPLE 5 : Crème démaquillante

On prépare une crème démaquillante en procédant au mélange des ingrédients suivants :

### A - Phase grasse

- Tétradécafluorohexane 10,0 %
- Cyclohexasiloxane 5,0 %

### B - Phase aqueuse

- Polymère d'AMPS vendu sous la dénomination "HOSTACERIN AMPS®" par la Société CLARIANT 2,0 %
- Conservateurs 0,2 %
- Glycérol 5,0 %
- Eau 77,8 %

Cette crème-gel sans tensioactif (ni émulsionnant, ni détergent) est particulièrement bien adaptée au démaquillage en douceur des peaux sensibles et délicates.

## Revendications

1. Utilisation pour le démaquillage ou le nettoyage de la peau du corps ou du visage, ou des lèvres, d'une composition anhydre ou aqueuse, caractérisée par le fait que ladite composition contient au moins un composé fluoré volatil, en une proportion d'au moins 0,5 % en poids par rapport au poids total de ladite composition.

2. Utilisation selon la revendication 1, caractérisée par le fait que ladite composition contient ledit composé fluoré volatil en une proportion d'au moins 5 %, de préférence entre 5 et 80 % et de préférence encore entre 8 et 70 % en poids par rapport au poids total de ladite composition.

3. Utilisation selon l'une des revendications précédentes, caractérisée par le fait que ledit composé fluoré volatil est choisi parmi, seuls ou en mélange :
1°) les perfluorocycloalkyles répondant à la formule (I) suivante : 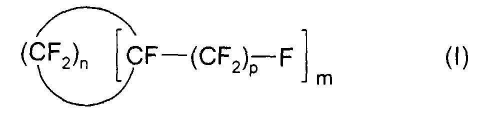 dans laquelle :
n est 3, 4 ou 5
m est 1 ou 2, et
p est 1,2 ou 3
sous réserve que lorsque m=2, les groupements ne sont pas nécessairement en alpha, l'un par rapport à l'autre;
2°) les perfluoroalcanes répondant à la formule (II) suivante :
CF₃-(CF₂)ₘ-CF₂X (II)
dans laquelle :
m est un entier compris entre 2 à 8, et
X représente Br ou F;
3°) les fluoroalkyles ou hétérofluoroalkyles répondant à la formule (III) suivante :
CH₃-(CH₂)ₙ-[Z]ₜ-X-CF₃ (III)
dans laquelle :
t est 0 ou 1,
n est 0, 1, 2 ou 3,
X est un radical perfluoroalkyle, linéaire ou ramifié, ayant de 2 à 5 atomes de carbone, et
Z est O, S ou NR, R étant un hydrogène, un radical -(CH₂)ₙ-CF₃ ou -(CF₂)ₘ-CF₃, m étant 2, 3, 4 ou 5; et
4°) les dérivés de perfluoromorpholine répondant à la formule (IV) suivante : 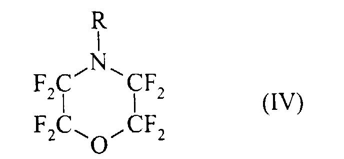 dans laquelle R est un radical perfluoroalkyle en C₁-C₄.

4. Utilisation selon l'une quelconque des revendications précédentes, caractérisée par le fait que le composé fluoré volatil est choisi dans le groupe constitué par le perfluorométhylcyclopentane, le perfluoro-1,3 diméthylcyclohexane, le perfluoro 1,2-diméthylcyclobutane, le dodécafluoropentane, le tétradécafluorohexane, le bromoperfluorooctyle, le nonafluorométhoxybutane, le nonafluoroéthoxyisobutane et la 4-trifluorométhyl perfluoromorpholine.

5. Utilisation selon l'une quelconque des revendications précédentes, caractérisée par le fait que ladite composition se présente sous forme d'une huile anhydre contenant le composé fluoré volatil à l'état soluble ou finement dispersé dans au moins une huile cosmétique.

6. Utilisation selon la revendication 5, caractérisée par le fait que le composé fluoré volatil est présent en une proportion de 20 à 80 % et de préférence entre 30 et 65 % par rapport au poids total de la composition.

7. Utilisation selon l'une quelconque des revendications 1 à 4, caractérisée par le fait que ladite composition se présente sous forme d'une émulsion eau-dans-l'huile ou huile-dans-l'eau, le composé fluoré volatil étant présent à l'état soluble ou dispersé dans la phase grasse de l'émulsion.

8. Composition cosmétique susceptible d'être utilisée pour le démaquillage ou le nettoyage de la peau du corps ou du visage, ou des lèvres, se présentant sous la forme d'une émulsion comprenant une phase grasse, une phase aqueuse et éventuellement un agent tensioactif, caractérisée par le fait que ladite phase grasse, contient au moins 5 % en poids d'un composé fluoré volatil, et est présente en une proportion comprise entre 2 et 70 % en poids, et que la phase aqueuse est présente en une proportion comprise entre 30 et 98 % en poids par rapport au poids total de la composition.

9. Composition selon la revendication 8, caractérisée par le fait que le composé fluoré volatil est présent dans ladite phase grasse en une proportion comprise entre 10 et 100 %, de préférence entre 30 et 95 % et de préférence encore entre 40 et 80 % en poids par rapport au poids de ladite phase.

10. Composition selon l'une des revendications 8 à 9, caractérisée par le fait que ladite phase grasse comprend en outre une huile cosmétique non fluorée miscible ou non miscible avec le composé fluoré volatil.

11. Procédé cosmétique de démaquillage ou de nettoyage de la peau du corps ou du visage, ou des lèvres comprenant les étapes consistant à utiliser, par application sur les parties de la peau à démaquiller ou à nettoyer, une quantité suffisante d'une composition anhydre ou aqueuse comprenant au moins un composé fluoré volatil en une proportion d'au moins 0,5 % en poids par rapport au poids total de ladite composition, à masser ensuite légèrement, et à éliminer ladite composition à l'aide d'un coton éventuellement imbibé d'eau.
